# FASCICULE DE BREVET EUROPEEN

(11) **EP 2 374 432 B1**
(45) Date de publication et mention de la délivrance du brevet: **28.08.2013**
(21) Numéro de dépôt: 11158185.6
(22) Date de dépôt: 15.03.2011
(51) Int. Cl.: A61F 2/32, A61F 2/34

(54) **Gamme d'implants cotyloïdiens de différentes tailles**
Reihe von Hüftpfannen mit unterschiedlicher Grö$e
Series of acetabular implants of different sizes

(30) Priorité: 08.04.2010 FR 1052639
(43) Date de publication de la demande: 12.10.2011
(73) Titulaire: Cartillier, Jean-Claude, 69008 Lyon (FR); Machenaud, Alain, 74330 La Balme de Sillingy (FR); Ait Si Selmi, Tarik, 69006 Lyon (FR); Rollier, Jean-Charles, 74370 Saint Martin Bellevue (FR); Bonnin, Michel, 69002 Lyon (FR); Jacquot, Laurent, 74370 Charvonnex (FR); Balay, Bruno, 01600 Trevoux (FR); Charlet, Claude, 69370 Saint Didier au Mont d'Or (FR); Fessy, Michel Henri, 69230 Saint Genis Laval (FR); Semay, Jean-Marc, 42270 Saint Priest en Jarez (FR); Setiey, Louis, 69400 Gleize (FR); Chatelet, Jean-Christophe, 01480 Jassans (FR); Vidalain, Jean-Pierre, 74940 Annecy le Vieux (FR)
(72) Inventeur: Cartillier, Jean-Claude, 69008 Lyon (FR); Machenaud, Alain, 74330 La Balme de Sillingy (FR); Ait Si Selmi, Tarik, 69006 Lyon (FR); Rollier, Jean-Charles, 74370 Saint Martin Bellevue (FR); Bonnin, Michel, 69002 Lyon (FR); Jacquot, Laurent, 74370 Charvonnex (FR); Balay, Bruno, 01600 Trevoux (FR); Charlet, Claude, 69370 Saint Didier au Mont d'Or (FR); Fessy, Michel Henri, 69230 Saint Genis Laval (FR); Semay, Jean-Marc, 42270 Saint Priest en Jarez (FR); Setiey, Louis, 69400 Gleize (FR); Chatelet, Jean-Christophe, 01480 Jassans (FR); Vidalain, Jean-Pierre, 74940 Annecy le Vieux (FR)
(74) Mandataire: Maureau, Philippe

(56) Documents cités:
- EP-A1- 0 821 922
- WO-A1-2009/118673
- FR-A1- 2 868 687
- FR-A1- 2 903 882

## Description

La présente invention concerne u n e gamme d'implants cotyloïdiens, et plus particulièrement une gamme d'implants cotyloïdiens dits « à double mobilité ».

Un implant cotyloïdien à double mobilité comporte, de façon connue, d'une part une cupule destinée à être fixée dans une cavité cotyloïdienne et comportant une partie polaire sensiblement hémisphérique et une partie équatoriale prolongeant la partie polaire, la partie polaire et la partie équatoriale délimitant une cavité intérieure sensiblement hémisphérique, et d'autre part un insert monté articulé dans la cavité intérieure délimitée par les parties polaire et équatoriale de la cupule, l'insert étant délimité extérieurement par une portion de surface sphérique externe et délimitant lui-même une cavité intérieure présentant une portion de surface sphérique interne et destinée au montage de manière articulée et rétentive d'une tête fémorale prothétique.

La présence d'une telle partie équatoriale permet de limiter les risques de sorties d'un insert hors de la cupule correspondante, et donc les risques de luxation de l'implant. Un tel implant cotyloïdien limite par conséquent les reprises chirurgicales.

Il doit cependant être noté que les risques de luxation restent importants lorsque la « jumping distance », c'est-à-dire la distance séparant le fond de la cupule et le bord délimitant l'ouverture de cette dernière, est faible, ce qui est normalement le cas pour les petites tailles d'implants.

Afin de limiter les risques de luxation pour les petites tailles d'implants, il est connu de prévoir une hauteur de partie équatoriale importante et constante pour l'ensemble des implants appartenant à une gamme de manière à assurer une « jumping distance » satisfaisante pour l'ensemble des implants.

Toutefois, la partie équatoriale de la cupule d'un tel implant cotyloïdien est susceptible, pour les grandes tailles d'implants, d'entrer en conflit avec des muscles ou des tendons situés à proximité du cotyle osseux, et notamment avec le psoas du patient, et donc de provoquer des douleurs chez ce dernier.

Afin de réduire les risques de luxation, il est également connu de décaler les centre des portions de surface sphérique externe et interne de l'insert de telle sorte que le centre de la portion de surface sphérique interne soit situé du côté du fond de la cavité intérieure délimité par l'insert. Ces dispositions permettent d'assurer un repositionnement optimal de l'insert par rapport à la cupule en position debout du patient, du fait du couple créé par les forces exercées sur la tête fémorale prothétique qui engendre la rotation de l'insert jusqu'à ce que celui-ci se trouve en position d'équilibre.

Il doit cependant être noté que le décalage entre ces deux centres peut ne pas être suffisant pour assurer un repositionnement optimal de l'insert pour des grandes tailles d'implants. En effet, plus l'implant est de grande taille, plus les frottements entre la surface extérieure de l'insert et la surface intérieure de la cupule sont importants et s'opposent de ce fait au couple créé par les forces exercées sur la tête fémorale prothétique et visant au replacement de l'insert, ce qui peut engendrer un risque de luxation de la tête fémorale prothétique et ainsi nuire à la fiabilité de l'implant.

La présente invention vise à remédier à ces inconvénients.

L'art antérieur le plus proche est divulgué par FR-A-2 868 687.

Le problème technique à la base de l'invention consiste donc à fournir une gamme d'implants cotyloïdiens qui soient de structure simple, économique et fiable, tout en permettant d'éviter les risques de luxation et de conflit avec l'organisme du patient.

A cet effet, l'invention concerne une gamme d'implants cotyloïdiens de différentes tailles du type comprenant :
- une cupule destinée à être fixée dans une cavité cotyloïdienne et comportant une partie polaire sensiblement hémisphérique et une partie équatoriale prolongeant la partie polaire, la partie polaire et la partie équatoriale délimitant une cavité intérieure sensiblement hémisphérique,
- un insert monté articulé dans la cavité intérieure délimitée par les parties polaire et équatoriale de la cupule, l'insert délimitant une cavité intérieure présentant une portion de surface sphérique interne et destinée au montage de manière articulée et rétentive d'une tête fémorale prothétique,
caractérisée en ce que la hauteur des parties équatoriales des cupules des différents implants cotyloïdiens appartenant à la gamme varie de manière décroissante en fonction de l'augmentation de la taille des implants cotyloïdiens.

Ainsi, la hauteur de la partie équatoriale de la cupule de l'implant de plus petite taille peut être définie importante afin de favoriser la stabilité des implants de petites tailles, sans que cela ne soit préjudiciable pour les implants grandes tailles. En effet, du fait de la diminution de la hauteur des parties équatoriales en fonction de l'augmentation de la taille des implants cotyloïdiens, la hauteur des parties équatoriales des cupules des implants de grandes tailles est faible de telle sorte que les cupules de ces derniers ne sont pas susceptibles d'engendrer des conflits avec l'organisme du patient, et notamment le psoas de ce dernier. Par conséquent, la gamme selon l'invention permet d'éviter les risques de luxation, tout évitant les risques de conflits avec l'organisme du patient.

De préférence, la hauteur des parties équatoriales des cupules des différents implants cotyloïdiens appartenant à la gamme décroit de manière homothétique ou par palier entre deux tailles successives d'implants cotyloïdiens.

Selon un mode de réalisation, la partie équatoriale de chaque cupule s'étend sur toute la périphérie de cette dernière.

Avantageusement, chaque insert est délimité extérieurement par une portion de surface sphérique externe, et le centre de la portion de surface sphérique interne de l'insert d'au moins l'un des implants cotyloïdiens est décalé par rapport au centre de la portion de surface sphérique externe dudit insert, en direction du fond de la cavité intérieure délimité par ledit insert. De préférence, les centres des portions de surface sphérique externe et interne de l'insert d'au moins l'un des implants cotyloïdiens sont décalés suivant l'axe de symétrie de l'insert.

De façon avantageuse, la distance entre le centre de la portion de surface sphérique externe de l'insert d'un implant cotyloïdien et le centre de la portion de surface sphérique interne dudit insert varie de manière croissante en fonction de l'augmentation de la taille des implants cotyloïdiens. Ces dispositions permettent d'assurer un repositionnement optimal de l'insert par rapport à la cupule pour l'ensemble des implants appartenant à la gamme, et plus particulièrement pour les implants de grandes tailles.

La distance entre le centre de la portion de surface sphérique externe de l'insert d'un implant cotyloïdien et le centre de la portion de surface sphérique interne dudit insert augmente par exemple de manière homothétique ou par palier entre deux tailles successives d'implants cotyloïdiens.

Selon un mode de réalisation, chaque cupule présente une surface intérieure lisse et polie.

Avantageusement, la cavité intérieure de chaque insert présente une portion de surface sphérique s'étendant sur plus d'une demi-sphère et prolongée en direction de l'ouverture de l'insert par une portion tronconique divergente.

De préférence, chaque cupule comprend, sur sa surface extérieure, des moyens d'ancrage osseux. Les moyens d'ancrage osseux comportent avantageusement un filetage s'étendant sur au moins une portion de la surface extérieure de la partie polaire, et de préférence également sur au moins une portion de la surface extérieure de la partie équatoriale.

Il convient de noter que les implants cotyloïdiens appartenant à la gamme peuvent être à fixation cimentée ou à fixation biologique.

De préférence, chaque insert est réalisé en polyéthylène, et chaque cupule est métallique, de préférence en acier inoxydable ou en un alliage de chrome-cobalt.

Avantageusement, la surface extérieure de chaque cupule est rugueuse et de préférence au moins une portion de la surface extérieure de la cupule est revêtue d'un revêtement de titane.

De façon préférentielle, la partie équatoriale de chaque cupule est délimitée intérieurement par une surface tronconique divergente en direction de l'ouverture de ladite cupule. Ces dispositions permettent de faciliter la réinsertion de l'insert dans la cupule en cas de luxation de ce dernier, et évite donc une réintervention chirurgicale. De plus, ces dispositions permettent d'augmenter l'amplitude de mouvement du col fémoral prothétique, tout en réduisant les risques de conflit entre ce dernier et la cupule.

De toute façon l'invention sera bien comprise à l'aide de la description qui suit en référence au dessin schématique annexé représentant, à titre d'exemple non limitatif, une forme d'exécution de cette gamme d'implants cotyloïdiens.
Figure 1 est une vue schématique en coupe de l'implant cotyloïdien de plus petite taille appartenant à la gamme selon l'invention.
Figure 2 est une vue schématique en coupe de l'implant cotyloïdien de plus grande taille appartenant à la gamme selon l'invention.
Figures 3 est une vue schématique en coupe de l'implant cotyloïdien de plus petite taille selon une variante de réalisation de l'invention.
Figure 4 est une vue schématique en coupe de l'implant cotyloïdien de la figure 1 illustrant l'amplitude de mouvement d'un col fémoral prothétique respectivement à l'intérieur de l'implant de la figure 1 et à l'intérieur de l'implant de la figure 3.

La gamme d'implants cotyloïdiens selon l'invention peut comporter par exemple six implants cotyloïdiens de tailles différentes, c'est-à-dire de différents diamètres. Les implants cotyloïdiens appartenant à la gamme peuvent être à fixation cimentée ou à fixation biologique.

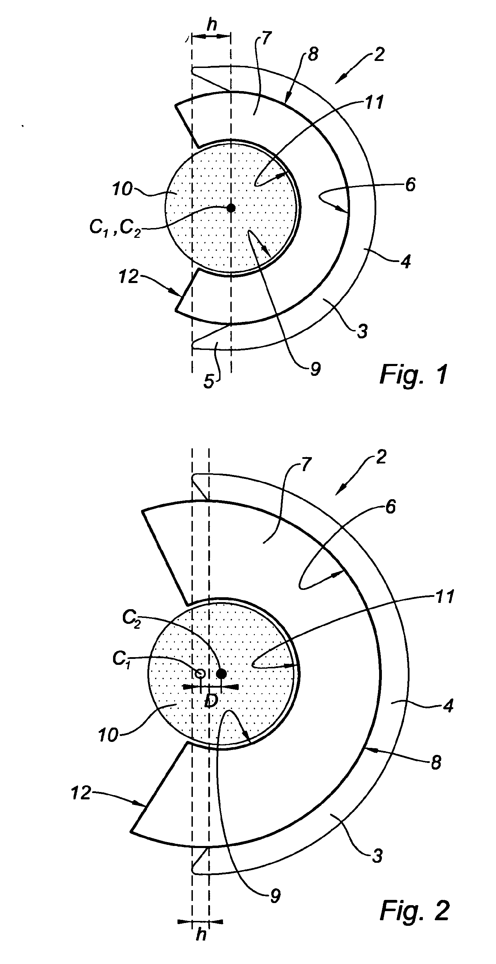

Comme montré sur la figure 1, chaque implant cotyloïdien 2 comprend une cupule métallique 3 destinée à être fixée dans une cavité cotyloïdienne d'un patient et comportant une partie polaire hémisphérique 4 et une partie équatoriale 5 prolongeant la partie polaire 4. La partie polaire 4 et la partie équatoriale 5 délimitent une cavité intérieure 6 sensiblement hémisphérique. La partie équatoriale 5 s'étend sur toute la périphérie de la cupule et délimite l'ouverture de cette dernière. La partie équatoriale 5 est avantageusement délimitée intérieurement par une surface tronconique divergente s'étendant de la partie polaire jusqu'à l'ouverture de la cupule. La partie équatoriale 5 est avantageusement délimitée extérieurement par une surface cylindrique.

De préférence, chaque cupule 3 comprend, sur sa surface extérieure, des moyens d'ancrage osseux (non représentés sur les figures). Les moyens d'ancrage osseux peuvent comporter par exemple un filetage ménagé sur la surface extérieure de la partie équatoriale 5.

Chaque cupule 3 est de préférence réalisée en acier inoxydable ou en un alliage de chrome-cobalt. Chaque cupule 3 présente avantageusement une surface intérieure lisse et polie et une surface extérieure rugueuse. De façon préférentielle, au moins une portion de la surface extérieure de chaque cupule 3 est revêtue d'un revêtement de titane.

Chaque implant cotyloïdien 2 comprend également un insert 7 réalisé en polyéthylène monté articulé dans la cavité intérieure 6 délimitée par les parties polaire et équatoriale 4, 5 de la cupule. Chaque insert 7 est délimité extérieurement par une portion de surface sphérique externe 8 et délimite lui-même une cavité intérieure 9 destinée au montage de manière articulée et rétentive d'une tête fémorale prothétique 10. La cavité intérieure 9 de chaque insert présente une portion de surface sphérique interne 11 s'étendant sur plus d'une demi-sphère et prolongée en direction de l'ouverture de l'insert par une portion tronconique divergente 12.

Comme montré sur la figure 1, le centre C₁ de la portion de surface sphérique externe 8 de l'insert de l'implant cotyloïdien 2 de plus petite taille est confondu avec le centre C₂ de la portion de surface sphérique interne 11 dudit insert.

Comme montré sur la figure 2, le centre C₂ de la portion de surface sphérique interne 11 de l'insert de l'implant cotyloïdien 2 de plus grande taille est décalé d'une distance D par rapport au centre C₁ de la portion de surface sphérique externe 8 dudit insert, suivant l'axe de symétrie de l'insert et en direction du fond de la cavité intérieure 9 délimité par ledit insert.

La hauteur h des parties équatoriales 5 des cupules 3 des différents implants cotyloïdiens 2 appartenant à la gamme varie de manière décroissante en fonction de l'augmentation de la taille des implants cotyloïdiens. De préférence, la hauteur des parties équatoriales 5 des cupules 3 des différents implants cotyloïdiens 2 appartenant à la gamme décroit de manière homothétique ou par palier entre deux tailles successives d'implants cotyloïdiens. Il convient de noter que la hauteur h est maximale pour l'implant cotyloïdien de plus petite taille et minimale pour l'implant cotyloïdien de plus grande taille.

De façon avantageuse, la distance D entre le centre C₁ de la portion de surface sphérique externe 8 de l'insert d'un implant cotyloïdien et le centre C₂ de la portion de surface sphérique interne 11 dudit insert varie de manière croissante en fonction de l'augmentation de la taille des implants cotyloïdiens. La distance D entre le centre C₁ de la portion de surface sphérique externe 8 de l'insert d'un implant cotyloïdien et le centre C₂ de la portion de surface sphérique interne 11 dudit insert augmente par exemple de manière homothétique ou par palier entre deux tailles successives d'implants cotyloïdiens. Il convient de noter que la distance D est nulle pour l'implant cotyloïdien de plus petite taille et maximale pour l'implant cotyloïdien de plus grande taille.

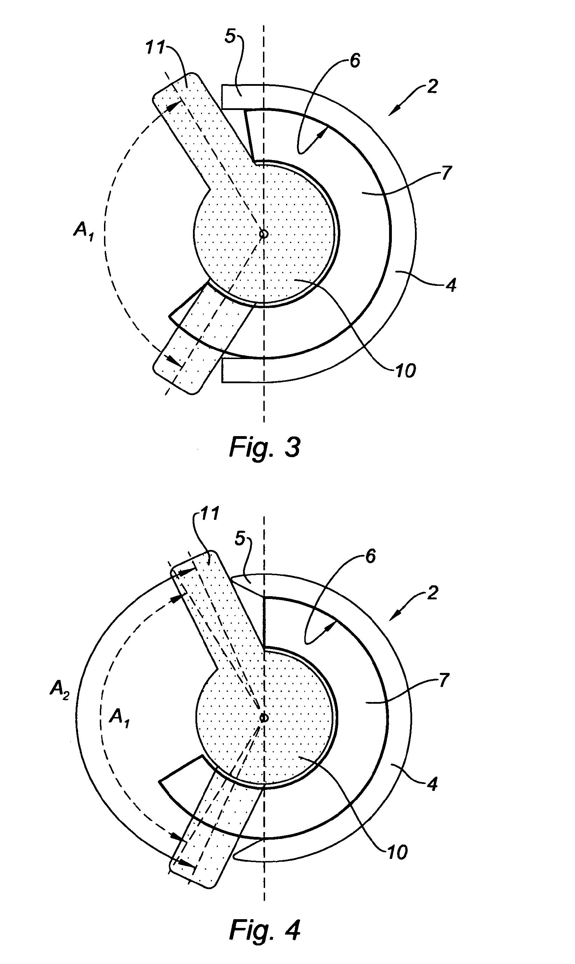

La figure 3 représente un implant cotyloïdien 2 qui diffère de celui représenté sur la figure 1 essentiellement en ce que la partie équatoriale 5 est cylindrique. L'amplitude de mouvement A₁ d'un col fémoral prothétique 11 à l'intérieur de l'implant cotyloïdien 2 est montré sur la figure 3 en illustrant les deux positions extrêmes du col fémoral prothétique 11.

La figure 4 illustre les amplitudes de mouvement A₁ et A₂ d'un col fémoral prothétique 11 respectivement à l'intérieur de l'implant cotyloïdien de la figure 3 et à l'intérieur de l'implant cotyloïdien de la figure 1. Comme montré sur la figure 4, le fait de délimiter intérieurement la partie équatoriale 5 de chaque cupule par une surface tronconique divergente en direction de l'ouverture de ladite cupule permet d'augmenter l'amplitude de mouvement du col fémoral prothétique 11 à l'intérieur de l'implant cotyloïdien.

Comme il va de soi, l'invention ne se limite pas à la seule forme d'exécution de cette gamme d'implants cotyloïdiens, décrite ci-dessus à titre d'exemple, elle en embrasse au contraire toutes les variantes de réalisation.

## Revendications

1. Gamme d'implants cotyloïdiens (2) de différentes tailles du type comprenant :
- une cupule (3) destinée à être fixée dans une cavité cotyloïdienne et comportant une partie polaire (4) sensiblement hémisphérique et une partie équatoriale (5) prolongeant la partie polaire, la partie polaire et la partie équatoriale délimitant une cavité intérieure (6) sensiblement hémisphérique,
- un insert (7) monté articulé dans la cavité intérieure délimitée par les parties polaire et équatoriale de la cupule, l'insert délimitant une cavité intérieure (9) présentant une portion de surface sphérique interne (11) et destinée au montage de manière articulée et rétentive d'une tête fémorale prothétique,
**caractérisée en ce que** la hauteur des parties équatoriales (5) des cupules (3) des différents implants cotyloïdiens (2) appartenant à la gamme varie de manière décroissante en fonction de l'augmentation de la taille des implants cotyloïdiens.

2. Gamme selon la revendication 1, **caractérisée en ce que** la hauteur des parties équatoriales (5) des cupules (3) des différents implants cotyloïdiens (2) appartenant à la gamme décroit de manière homothétique ou par palier entre deux tailles successives d'implants cotyloïdiens.

3. Gamme selon la revendication 1 ou 2, **caractérisée en ce que** chaque insert (7) est délimité extérieurement par une portion de surface sphérique externe (8), et **en ce que** le centre (C₂) de la portion de surface sphérique interne de l'insert d'au moins l'un des implants cotyloïdiens est décalé par rapport au centre (C₁) de la portion de surface sphérique externe (11) dudit insert, en direction du fond de la cavité intérieure (9) délimité par ledit insert.

4. Gamme selon la revendication 3, **caractérisée en ce que** les centres (C₁, C₂) des portions de surface sphérique externe et interne (8, 11) de l'insert (7) d'au moins l'un des implants cotyloïdiens sont décalés suivant l'axe de symétrie de l'insert.

5. Gamme selon la revendication 3 ou 4, **caractérisée en ce que** la distance (D) entre le centre (C₁) de la portion de surface sphérique externe (8) de l'insert (7) d'un implant cotyloïdien (2) et le centre (C₂) de la portion de surface sphérique interne (11) dudit insert varie de manière croissante en fonction de l'augmentation de la taille des implants cotyloïdiens.

6. Gamme selon la revendication 5, **caractérisée en ce que** la distance (D) entre le centre (C₁) de la portion de surface sphérique externe (8) de l'insert (7) d'un implant cotyloïdien et le centre (C₂) de la portion de surface sphérique interne (11) dudit insert augmente de manière homothétique ou par palier entre deux tailles successives d'implants cotyloïdiens.

7. Gamme selon l'une des revendications 1 à 6, **caractérisée en ce que** chaque cupule (3) présente une surface intérieure lisse et polie.

8. Gamme selon l'une des revendications 1 à 7, **caractérisée en ce que** la cavité intérieure (9) de chaque insert (7) présente une portion de surface sphérique (11) s'étendant sur plus d'une demi-sphère et prolongée en direction de l'ouverture de l'insert par une portion tronconique divergente (12).

9. Gamme selon l'une des revendications 1 à 8, **caractérisée en ce que** chaque cupule (3) comprend, sur sa surface extérieure, des moyens d'ancrage osseux.

10. Gamme selon l'une des revendications 1 à 9, **caractérisée en ce que** la partie équatoriale (5) de chaque cupule (3) est délimitée intérieurement par une surface tronconique divergente en direction de l'ouverture de ladite cupule.

## Patentansprüche

1. Sortiment von Gelenkpfannenimplantaten (2) verschiedener Größen des Typs, der umfasst:
- einen Becher (3), der zur Befestigung in einer Gelenkpfannenkavität bestimmt ist und einen etwa halbkugeligen polaren Teil (4) und einen äquatorialen Teil (5), der den polaren Teil verlängert, aufweist, wobei der polare Teil und der äquatoriale Teil eine etwa halbkugelige innere Kavität (6) begrenzen,
- einen Einsatz (7), der gelenkig in der inneren Kavität montiert ist, die vom polaren und äquatorialen Teil des Bechers begrenzt ist, wobei der Einsatz eine innere Kavität (9) begrenzt, die einen inneren kugeligen Oberflächenabschnitt (11) aufweist und zur gelenkigen und rückhaltenden Montage eines prothetischen Femurkopfes bestimmt ist,
**dadurch gekennzeichnet, dass** sich die Höhe der äquatorialen Teile (5) der Becher (3) der verschiedenen Gelenkpfannenimplantate (2), die zu dem Sortiment gehören, in Abhängigkeit von der Zunahme der Größe der Gelenkpfannenimplantate abfallend ändert.

2. Sortiment nach Anspruch 1, **dadurch gekennzeichnet, dass** die Höhe der äquatorialen Teile (5) der Becher (3) der verschiedenen Gelenkpfannenimplantate (2), die zu dem Sortiment gehören, homothetisch oder stufig zwischen zwei aufeinanderfolgenden Größen von Gelenkpfannenimplantaten abnimmt.

3. Sortiment nach Anspruch 1 oder 2, **dadurch gekennzeichnet, dass** jeder Einsatz (7) außen von einem externen kugeligen Oberflächenabschnitt (7) begrenzt ist und dass das Zentrum (C₂) des inneren kugeligen Oberflächenabschnitts mindestens eines der Gelenkpfannenimplantate im Verhältnis zum Zentrum (C₁) des externen kugeligen Oberflächenabschnitts (11) des Einsatzes in Richtung des Bodens der inneren Kavität (9), der von dem Einsatz begrenzt wird, versetzt ist.

4. Sortiment nach Anspruch 3, **dadurch gekennzeichnet, dass** die Zentren (C₁, C₂) des externen und internen kugeligen Oberflächenabschnitts (8, 11) des Einsatzes mindestens eines der Gelenkpfannenimplantate gemäß der Symmetrieachse des Einsatzes versetzt sind.

5. Sortiment nach Anspruch 3 oder 4, **dadurch gekennzeichnet, dass** sich der Abstand (D) zwischen dem Zentrum (C₁) des externen kugeligen Oberflächenabschnitts (8) des Einsatzes (7) eines Gelenkpfannenimplantats und dem Zentrum (C₂) des internen kugeligen Oberflächenabschnitts (11) des Einsatzes in Abhängigkeit von der Zunahme der Größe der Gelenkpfannenimplantate zunehmend ändert.

6. Sortiment nach Anspruch 5, **dadurch gekennzeichnet, dass** der Abstand (D) zwischen dem Zentrum (C₁) des externen kugeligen Oberflächenabschnitts (8) des Einsatzes (7) eines Gelenkpfannenimplantats und dem Zentrum (C₂) des internen kugeligen Oberflächenabschnitts (11) des Einsatzes homothetisch oder stufig zwischen zwei aufeinanderfolgenden Größen von Gelenkpfannenimplantaten zunimmt.

7. Sortiment nach einem der Ansprüche 1 bis 6, **dadurch gekennzeichnet, dass** jeder Becher (3) eine glatte und polierte innere Oberfläche aufweist.

8. Sortiment nach einem der Ansprüche 1 bis 7, **dadurch gekennzeichnet, dass** die innere Kavität (9) jedes Einsatzes (7) einen kugeligen Oberflächenabschnitt (11) aufweist, der sich über mehr als eine Halbkugel erstreckt und in Richtung der Öffnung des Einsatzes durch einen divergierenden kegelstumpfförmigen Abschnitt (12) verlängert wird.

9. Sortiment nach einem der Ansprüche 1 bis 8, **dadurch gekennzeichnet, dass** jeder Becher (3) auf seiner äußeren Oberfläche Knochenverankerungsmittel umfasst.

10. Sortiment nach einem der Ansprüche 1 bis 9, **dadurch gekennzeichnet, dass** der äquatoriale Teil (5) jedes Bechers (3) innen von einer in Richtung der Öffnung des Bechers divergierenden kegelstumpfförmigen Oberfläche begrenzt wird.

## Claims

1. A line of acetabular implants (2) of different sizes, of the type comprising:
- a cup (3) designed to be fastened in an acetabular cavity and including a substantially hemispherical polar part (4) and an equatorial part (5) extending the polar part, the polar part and the equatorial part delimiting a substantially hemispherical inner cavity (6),
- an insert (7) articulately mounted in the inner cavity delimited by the polar and equatorial parts of the cup, the insert delimiting an inner cavity (9) having an inner spherical surface portion (11) and designed to mount a prosthetic femoral head in an articulated and retentive manner,
**characterized in that** the heights of the equatorial parts (5) of the cups (3) of the various acetabular implants (2) of the line varies decreasing as the size of the acetabular implants increases.

2. The line according to claim 1, **characterized in that** the height of the equatorial parts (5) of the cups (3) of the various acetabular implants (2) of the line decreases in a homothetic manner or by plateau between two successive sizes of acetabular implants.

3. The line according to claim 1 or 2, **characterized in that** each insert (7) is outwardly delimited by an outer spherical surface portion (8), and **in that** the center (C₂) of the inner spherical surface portion of the insert of at least one of the acetabular implants is offset with respect to the center (C₁) of the outer spherical surface portion (11) of said insert, toward the bottom of the inner cavity (9) delimited by said insert.

4. The line according to claim 3, **characterized in that** the centers (C₁, C₂) of the outer and inner spherical surface portions (8, 11) of the insert (7) of at least one of the acetabular implants are offset along the axis of symmetry of the insert.

5. The line according to claim 3 or 4, **characterized in that** the distance (D) between the center (C₁) of the outer spherical surface portion (8) of the insert (7) of an acetabular implant (2) and the center (C₂) of the inner spherical surface portion (11) of said insert varies increasing as the size of the acetabular implants increases.

6. The line according to claim 5, **characterized in that** the distance (D) between the center (C₁) of the outer spherical portion (8) of the insert (7) of an acetabular implant and the center (C₂) of the inner spherical surface portion (11) of said insert increases in a homothetic manner or by plateau between two successive sizes of acetabular implants.

7. The line according to one of claims 1 to 6, **characterized in that** each cup (3) has a smooth and polished inner surface.

8. The line according to one of claims 1 to 7, **characterized in that** the inner cavity (9) of each insert (7) has a spherical surface portion (11) extending over more than a half-sphere and extended towards the opening of the insert by a diverging tapered portion (12).

9. The line according to one of claims 1 to 8, **characterized in that** each cup (3) comprises bone anchoring means on its outer surface.

10. The line according to one of claims 1 to 9, **characterized in that** the equatorial part (5) of each cup (3) is inwardly delimited by a tapered surface diverging toward the opening of said cup.
